# EUROPEAN PATENT APPLICATION

(11) **EP 2 085 097 A1**
(43) Date of publication of application: **05.08.2009**
(21) Application number: 07828623.4
(22) Date of filing: 27.09.2007
(51) Int. Cl.: A61K 47/26, A61K 9/14, A61K 31/047, A61K 31/122, A61K 31/355, A61K 47/28, A61K 47/36, A61K 47/38, A61K 47/40, A61K 47/42

(54) **HIGHLY WATER-DISPERSIBLE POWDER AND METHOD OF PRODUCING THE SAME**

(30) Priority: 16.10.2006 JP 2006281323
(71) Applicant: Freund Corporation, Tokyo 163-6034 (JP)
(72) Inventor: YASUMI, Hirotsune, Tokyo 1636034 (JP); OHMAE, Masaru, Saitama-shi Saitama 3360027 (JP)
(74) Representative: Eisenführ, Speiser & Partner
(86) International application number: PCT/JP2007/068877
(87) International publication number: WO 2008/047559

(57) **Abstract**

Provided is a highly water dispersible powder which exhibits a high water dispersibility in spite of the inclusion of a poorly water soluble component, and maintains the high water dispersibility stably over time.

The highly water dispersible powder includes a poorly water soluble component, a water soluble polymer having a protective colloid effect, and a saponin. The highly water dispersible powder can be produced by a method including: emulsifying a poorly water soluble component in an aqueous solvent under the presence of a water soluble polymer having a protective colloid effect and a saponin, thereby preparing an emulsion; and drying the obtained emulsion.

## Description

### TECHNICAL FIELD

The present invention relates to a highly water dispersible powder and a production method thereof, the highly water dispersible powder containing a poorly water soluble component such as ubiquinones and fat soluble vitamins, and is suitably added to supplements, health foods, or the like.
Priority is claimed on Japanese Patent Application No. 2006-281323; filed October 16, 2006, the content of which is incorporated herein by reference.

### BACKGROUND ART

The ubiquinones are known to belong to a group of coenzymes collectively called coenzyme Q, and are widely distributed in the living world. Among these ubiquinones, coenzyme Q₁₀ (hereinafter referred to as CoQ₁₀) having 10 isoprene units, in particular, is widely used not only in drugs for heart disease or the like but also in the food industries concerning supplements, health foods or the like in recent years.
However, since CoQ₁₀ is poorly soluble in water, it is difficult to add it to food products, and this problem tends to limit the use thereof. Moreover, fat soluble vitamins such as vitamin E or carotenoids such as lutein which are poorly soluble in water also have the same problem.

Accordingly, in order to ease the addition of such a poorly water soluble component to food products, attempts have been made to form an emulsified material in which fine particles of a poorly water soluble component are dispersed in an aqueous solvent, by using fatty acid esters having surface activity, for example, glycerin fatty acid esters (refer to Patent Document 1 (Claim 4 or the like), and Patent Documents 2 and 3, for example).
However, such a material in an emulsified form is still unsatisfactory in terms of easy addition thereof to food products. Therefore, such a material is required to be made into a form of a highly water dispersible powder which is easier to add. As a method of obtaining a highly water dispersible powder, a method of drying an emulsified material obtained by the above-mentioned manner to remove a solvent may also be a possible option. However, when powdering a poorly water soluble component using glycerin fatty acid esters, glycerin fatty acid esters with a high hydrophilic-lipophilic balance (HLB) are generally used, and these glycerin fatty acid esters with a high HLB are usually waxy or viscous at normal temperatures. Accordingly, if a solvent is removed from the above-mentioned emulsified material, the resulting material is either a sticky powder with poor fluidity or a material in a non-powder form.

As a technique for powdering a poorly water soluble component by using a substance other than the glycerin fatty acid esters, a method is disclosed in which an emulsified material, where a poorly soluble material is dispersed in a solution of a water-soluble material, is first prepared, and a powder is obtained by removing water from this emulsified material (refer to Patent Document 4 (Claim 2 or the like), for example). In addition, a method of powdering a poorly water soluble material by using a water-soluble material and the glycerin fatty acid esters at the same time has also been studied. For example, Patent Documents 5, 6 (Claim 8 or the like), and 7 (Claim 8 or the like) have disclosed some methods with the aim of enhancing body absorption.
Also, a method of powdering a poorly water soluble material by using saponins, and also using glycerin fatty acid esters at the same time in some cases, has also been studied (refer to Patent Document 8, for example).
[Patent Document 1] Japanese Unexamined Patent Application, First Publication No. 2003-238396
[Patent Document 2] Japanese Unexamined Patent Application, First Publication No. 2004-196781
[Patent Document 3] Japanese Unexamined Patent Application, First Publication No. 2005-139122
[Patent Document 4] Japanese Unexamined Patent Application, First Publication No. 2003-313145
[Patent Document 5] Japanese Unexamined Patent Application, First Publication No. Sho 59-161314
[Patent Document 6] Japanese Unexamined Patent Application, First Publication No. Sho 58-13508
[Patent Document 7] Japanese Unexamined Patent Application, First Publication No. Sho 58-77810
[Patent Document 8] Japanese Unexamined Patent Application, First Publication No. Sho 60-64919

### DISCLOSURE OF THE INVENTION

### PROBLEMS TO BE SOLVED BY THE INVENTION

However, the powder obtained by removing water from an emulsified material, in which a water-soluble material is used, has unsatisfactory water dispersibility in spite of its powdery form. As a result, it was still difficult to add to food products, and the use thereof was limited in many cases.
In addition, when a poorly water soluble material and the glycerin fatty acid esters are used at the same time, the water dispersibility of the obtained powder tends to deteriorate over time. This is possibly due to the different modes of emulsifying action of the two components; that is, the emulsifying action of the poorly water soluble material is for forming protective colloids whereas the emulsifying action of the glycerin fatty acid esters is for micelle formation.
Moreover, the powder obtained by using glycerin fatty acid esters has problems in stability, such as the development of oxidative rancidity due to the oxidation of glycerin fatty acid esters over time.
Furthermore, even in cases where the saponins alone are used or the saponins and glycerin fatty acid esters are used at the same time, it has been difficult to obtain a powder having satisfactory water dispersibility and maintaining the dispersibility stably over time.

The present invention is made in view of the above circumstances and its object is to provide a highly water dispersible powder and a production method thereof, the powder exhibiting a high dispersibility in water (high water dispersibility) in spite of the inclusion of a poorly water soluble component and maintaining the high water dispersibility stably over time.

### MEANS FOR SOLVING THE PROBLEMS

As a result of intensive and extensive studies, the present inventors have found that a highly water dispersible powder having a high water dispersibility and maintaining the high water dispersibility stably over time can be provided by using a water soluble polymer having a protective colloid effect and a saponin at the same time, so as to complete the present invention.
The highly water dispersible powder of the present invention includes a poorly water soluble component, a water soluble polymer having a protective colloid effect, and a saponin.
The amount of a water soluble polymer having a protective colloid effect is preferably within the range of 5 to 500 parts by mass, more preferably within the range of 15 to 200 parts by mass, relative to 100 parts by mass of a poorly water soluble component. The amount of a saponin is preferably within the range of 1 to 50 parts by mass, more preferably within the range of 4 to 30 parts by mass, relative to 100 parts by mass of a poorly water soluble component.
The method of producing a highly water dispersible powder of the present invention includes: emulsifying a poorly water soluble component in an aqueous solvent under the presence of a water soluble polymer having a protective colloid effect and a saponin, thereby preparing an emulsion; and drying the emulsion.

### EFFECTS OF THE INVENTION

According to the present invention, a highly water dispersible powder can be provided which exhibits a high water dispersibility in spite of the inclusion of a poorly water soluble component, and maintains the high water dispersibility stably over time.

### BEST MODE FOR CARRYING OUT THE INVENTION

The present invention will be described in detail below.
The highly water dispersible powder of the present invention is a powder containing a poorly water soluble component, a water soluble polymer having a protective colloid effect, and a saponin, and one that exhibits a stable high water dispersibility over time.
As a poorly water soluble component, drugs may be mentioned as examples, and specific examples thereof include ubiquinones, fat soluble vitamins, carotenoids and refined fish oils. Among these poorly water soluble components, CoQ₁₀, vitamin E, and lutein are suitably used, because they are added to a broad range of food products such as supplements and health foods, and CoQ₁₀ in particular is suitably used. Further, a poorly water soluble component may be a material obtained by dispersing or dissolving the above substances in an oil phase that is in a liquid state at normal temperatures. When producing a highly water dispersible powder, it is preferable that a poorly water soluble component be in an oily and dispersed state in an aqueous solvent, although the details thereof will be described later. For this reason, as a poorly water soluble component, it is preferable to use either a component having a melting point equal to or less than the boiling point of water, preferably equal to or less than 95°C or, as mentioned earlier, a material obtained by dispersing or dissolving the above substances in an oil phase that is in a liquid state at normal temperatures.

A water soluble polymer having a protective colloid effect (hereinafter referred to as a protective colloid polymer) means a water soluble polymer that can act as a protective colloid. In the present description and claims, a protective colloid refers to a hydrophilic colloid that can surround the surface of a hydrophobic colloid and make the surface hydrophilic. In addition, in the present description and claims, the term "polymer" refers to a polymer or an oligomer formed by the polymerization of one or more kinds of monomers.
Such protective colloid polymers are not particularly limited as long as they are edible, and examples thereof include cellulose derivatives such as methylcellulose, hydroxyethylcellulose, hydroxypropylcellulose, hydroxypropylmethylcellulose, and sodium carboxymethylcellulose; and natural polymer materials such as gum arabic, tragacanth gum, and gelatin. One or more of these materials may be used as a protective colloid polymer.
Since the starch-based water soluble polymers such as dextrin and cyclodextrin, saccharides, glycerin fatty acid esters, and the like do not have a protective colloid effect (ability), these materials are not effective as a protective colloid polymer in the present invention. However, it should be noted that the starch-based water soluble polymers, saccharides and the like (wherein, glycerin fatty acid esters are not included) hardly inhibit the protective colloid effect of protective colloid polymers, and thus they may be used as an excipient described later.

As the saponins, triterpene-based saponins and steroidal saponins may be used. Specific examples thereof include quillaja saponin, yucca saponin, enju saponin, soybean saponin, enzymatically modified soybean saponin, tea seed saponin, and beet saponin. Of these, it is preferable to use at least one of quillaja saponin and yucca saponin, and it is more preferable to use quillaja saponin, since the use thereof results in the production of a highly water dispersible powder exhibiting even higher water dispersibility. Needless to say, two or more of these saponins may be used in combination.

In the aforementioned highly water dispersible powder, an optional component may be contained if necessary, in addition to the above-mentioned poorly water soluble component, protective colloid polymer, and saponins. As an optional component, various additives such as an excipient, a stabilizer of a poorly water soluble component, a perfume, and a coloring agent may be used. The excipient is preferably a water soluble powder, although there is no particular limitation. For example, one or more of starch-based water soluble polymers such as dextrin and cyclodextrin, sugar alcohols such as mannitol, and saccharides such as lactose can be used depending on the purpose. As a stabilizer, when a poorly water soluble component is CoQ₁₀, an organic acid such as malic acid is preferably used.

There is no particular limitation on the ratio of each component in the highly water dispersible powder. However, in order to provide a highly water dispersible powder having even higher water dispersibility and maintaining the high water dispersibility stably over time, it is preferable that a protective colloid polymer be contained within the ratio of 5 to 500 parts by mass, more preferably within the ratio of 15 to 200 parts by mass, relative to 100 parts by mass of the poorly water soluble component. In addition, it is preferable that a saponin be contained within the ratio of 1 to 50 parts by mass, more preferably within the ratio of 4 to 30 parts by mass, relative to 100 parts by mass of the poorly water soluble component.
Further, the content of the poorly water soluble component is preferably within the range of 1 to 70% by mass, more preferably within the range of 5 to 50% by mass, relative to 100% by mass of the highly water dispersible powder.

Such a highly water dispersible powder can be produced by a method including an emulsification step of emulsifying a poorly water soluble component in an aqueous solvent under the presence of a water soluble polymer having a protective colloid effect and a saponin, thereby preparing an emulsion; and a drying step of drying the obtained emulsion.
In the emulsification step, since it is preferable that a poorly water soluble component be in an oily and dispersed state in an aqueous solvent, it is preferable to prepare an emulsion at a temperature equal to or more than the melting point of the poorly water soluble component. The following specifically describes one example of such processes. First, an aqueous solvent is prepared in which temperature is controlled so as to achieve a temperature equal to or more than the melting point of a poorly water soluble component. A protective colloid polymer and a saponin are then added and dissolved therein. Subsequently, a poorly water soluble component is added thereto and mixed while maintaining the temperature. The resulting mixed liquid is then passed through a homogenizer, preferably a high pressure homogenizer. Thereby, a fine and homogeneous emulsion is obtained in which a poorly water soluble component is emulsified by the action of a protective colloid polymer and a saponin (emulsification step).
When using a material obtained by dispersing or dissolving ubiquinones, fat soluble vitamins, carotenoids, refined fish oil, or the like in an oil phase that is in a liquid state at normal temperatures as a poorly water soluble component, it is possible to prepare an emulsion at normal temperatures.

As an aqueous solvent, water such as purified water is usually used, and ethyl alcohol or the like may be used in combination in some cases. The concentration of the emulsion prepared in the emulsification step is not particularly limited and may be set appropriately depending on the type of a poorly water soluble drug. However, the concentration that can achieve a fine and homogeneous emulsion is preferable. In addition, the amount of a protective colloid polymer and a saponin may be such that the amount, relative to the amount of a poorly water soluble component, achieves the ratio described previously.

After the emulsification step, the obtained emulsion is introduced to a fluidized bed granulator, a spray dryer, or the like to dry, thereby obtaining a highly water dispersible powder (drying step).
It should be noted that even though there is a case where a granular material is obtained when using a fluidized bed granulator, such a granular material is also regarded as a powder in the present description.

If an excipient is contained in a highly water dispersible powder, the excipient may be added in either the emulsification step or the drying step. Specifically, when the drying step is carried out using a fluidized bed granulator, a method is preferable in which an excipient is made to flow inside the device in advance and then the emulsion obtained in the emulsification step is sprayed thereto. When the drying step is carried out using a spray dryer, an excipient may be added and dissolved in an aqueous solvent together with a protective colloid polymer and a saponin to prepare an emulsion containing an excipient, followed by the spray drying of the obtained emulsion.
When including an optional component such as a stabilizer of a poorly water soluble component, the optional component may be added to the emulsion.

All of the protective colloid polymer, saponin, and optional component may be used in the form of a liquid material where these components are dissolved or dispersed in water or an aqueous solvent in which a small amount of ethyl alcohol is added to water.

The highly water dispersible powder produced in such a manner exhibits a high water dispersibility in spite of the inclusion of a poorly water soluble component. Moreover, the high water dispersibility remains stable over time and the dispersibility in water hardly deteriorates. As a result, the highly water dispersible powder produced in such a manner is easily added to food products such as supplements and health foods, and thus can be used in a wide variety of applications. Specifically, not only the highly water dispersible powder can be provided to be added to food products such as health foods, but also the highly water dispersible powder can be sold directly as a health food or a supplement so that the consumer eventually ingests it directly or by dispersing it in water.

As described so far, according to the present invention, a highly water dispersible powder exhibiting a high water dispersibility that is stable over time can be provided. This is due to the combined use of a protective colloid polymer and a saponin.
That is, a saponin having a high micelle forming ability refines a poorly water soluble component, thereby incorporating the refined poorly water soluble component into the network structure of a protective colloid polymer more efficiently and more stably. It is thought that a high water dispersibility that is stable over time is developed as a result of the above incorporation. When no saponin is used, the obtained powder does not exhibit high water dispersibility that is stable over time. When a substance other than saponins which has a micelle forming ability (for example, glycerin fatty acid esters) is used, a poorly water soluble component is not incorporated in a protective colloid polymer efficiently and stably, possibly due to the emulsification mechanism that is different from that of saponins. For this reason, the obtained powder does not exhibit a high water dispersibility that is stable over time.
As described so far, according to the present invention, an excellent effect is achieved due to the combined use of a protective colloid polymer and a saponin causing a synergistic effect.

### [Examples]

### [Example 1]

12 g of gum arabic (manufactured by Ina Food Industry Co., Ltd. under the trade name of Inagel gum arabic A) and 4 g of quillaja saponin (manufactured by Maruzen Pharmaceuticals Co., Ltd. under the trade name of Quillajanin S-100 having a quillaja saponin content of 25% by mass) were dissolved in 50 g of purified water heated to about 60°C. Subsequently, while maintaining the temperature, 10 g of CoQ₁₀ (manufactured by Kougen Co., Ltd.) having a melting point of about 50°C was added and mixed, and the resulting mixture was then passed through a high pressure homogenizer twice at a processing pressure of 750 kg/cm², as a result of which a fine and homogeneous emulsion was obtained.
Then, by using a fluidized bed granulator (Model: FL-MINI manufactured by Freund Corporation), 77 g of dextrin (manufactured by Matsutani Chemical Industry Co., Ltd. under the trade name of Pineflow) was made to flow in the fluidized bed and the obtained emulsion was sprayed thereto, as a result of which an orange colored, highly water dispersible powder containing 10% by mass of CoQ₁₀ was obtained which was a mixture of powdery and granular forms.
The mass ratio of each of the components in this working Example was such that poorly water soluble component (CoQ₁₀) : protective colloid polymer (gum arabic) : saponin (quillaja saponin) = 100 : 120 : 10.

### [Example 2]

5 g of gelatin (manufactured by Maruha Corporation under the trade name of Jellice), 5 g of yucca saponin (manufactured by Maruzen Pharmaceuticals Co., Ltd. under the trade name of Sarakeep ALS having a yucca saponin content of 20% by mass), and 2 g of malic acid (manufactured by Fuso Chemical Co., Ltd. under the trade name of Malic Acid Fuso) were dissolved in 60 g of purified water heated to about 60°C. Subsequently, while maintaining the temperature, 10 g of the same type of CoQ₁₀ used in Example 1 was added and mixed, and the resulting mixture was then passed through a high pressure homogenizer twice at a processing pressure of 750 kg/cm², as a result of which a fine and homogeneous emulsion was obtained.
Then, by using the same fluidized bed granulator used in Example 1, 82 g of the same type of dextrin used in Example 1 was made to flow in the fluidized bed and the obtained emulsion was sprayed thereto, as a result of which an orange colored, highly water dispersible powder containing 10% by mass of CoQ₁₀ was obtained which was a mixture of powdery and granular forms.
The mass ratio of each of the components in this working Example was such that poorly water soluble component (CoQ₁₀) : protective colloid polymer (gelatin) : saponin (yucca saponin) = 100 : 50 : 10.

### [Example 3]

5 g of hydroxypropylcellulose (manufactured by Nippon Soda Co., Ltd. under the trade name of Celny-L, hereinafter referred to as HPC) and 10 g of the same type of quillaja saponin used in Example 1 were dissolved in 160 g of purified water at a normal temperature. Subsequently, 20 g of vitamin E (manufactured by Tama Biochemical Co., Ltd. under the trade name of E-MIX-α-1000), which is in a liquid state at a normal temperature, was added and mixed, and the resulting mixture was then passed through a high pressure homogenizer twice at a processing pressure of 500 kg/cm² while cooling, as a result of which a fine and homogeneous emulsion was obtained.
Then, by using the same fluidized bed granulator used in Example 1, 72.5 g of the same type of dextrin used in Example 1 was made to flow in the fluidized bed and the obtained emulsion was sprayed thereto, as a result of which a pale yellowish white colored, highly water dispersible powder containing 20% by mass of vitamin E was obtained which was a mixture of powdery and granular forms.
The mass ratio of each of the components in this working Example was such that poorly water soluble component (vitamin E) : protective colloid polymer (HPC) : saponin (quillaja saponin) = 100 : 25 : 12.5.

### [Example 4]

560 g of the same type of gum arabic used in Example 1 and 160 g of quillaja saponin (manufactured by Maruzen Pharmaceuticals Co., Ltd. under the trade name of Quillajanin C-100 having a quillaja saponin content of 25% by mass) were dissolved in 3,000 g of purified water heated to about 60°C. Subsequently, while maintaining the temperature, 400 g of the same type of CoQ₁₀ used in Example 1 was added and mixed, and the resulting mixture was then passed through a high pressure homogenizer twice at a processing pressure of 750 kg/cm², as a result of which a fine and homogeneous emulsion was obtained.
Then, by using a fluidized bed granulator (Model: FL-5 manufactured by Freund Corporation), 1,500 g of the same type of dextrin used in Example 1 and 1,500 g of mannitol (manufactured by Nikken Chemical and Synthetic Industry Co., Ltd. under the trade name of Mannitol Nikken) were made to flow in the fluidized bed and the obtained emulsion was sprayed thereto, as a result of which an orange colored, highly water dispersible powder containing 10% by mass of CoQ₁₀ was obtained which was a mixture of powdery and granular forms.
The mass ratio of each of the components in this working Example was such that poorly water soluble component (CoQ₁₀) : protective colloid polymer (gum arabic) : saponin (quillaja saponin) = 100 : 140 : 10.

### [Example 5]

100 g of the same type of HPC used in Example 3, 100 g of the same type of quillaja saponin used in Example 1, and 675 g of β-cyclodextrin (manufactured by Ensuiko Sugar Refining Co., Ltd. under the trade name of Dexpearl β-100) were dissolved in 2,000 g of purified water at a normal temperature. Then, in the obtained solution, 200 g of a lutein formulation (manufactured by Koyo Mercantile Co., Ltd. under the trade name of Flora GLO, a suspension of 20% by mass of lutein) in which lutein was dispersed and suspended in an oil phase and which was in a liquid form at a normal temperature was dispersed. The resultant was then emulsified using a homogenizer (4,000 rpm, 5 minutes), and then passed through a high pressure homogenizer twice at a processing pressure of 500 kg/cm², as a result of which a fine and homogeneous emulsion was obtained.
Subsequently, the obtained emulsion was spray dried using a spray dryer (Model: L-8 manufactured by Ohkawara Kakohki Co., Ltd.) with an inlet temperature of 170°C and an outlet temperature of 110°C, as a result of which a pale brownish white colored, highly water dispersible powder containing 20% by mass of lutein formulation was obtained.
The mass ratio of each of the components in this working Example was such that poorly water soluble component (lutein) : protective colloid polymer (HPC) : saponin (quillaja saponin) = 100 : 50 : 12.5.

### [Example 6]

250 g of the same type of gum arabic used in Example 1, 150 g of the same type of quillaja saponin used in Example 1, 112.5 g of the same type of β-cyclodextrin used in Example 5, and 200 g of the same type of mannitol used in Example 4 were dissolved in 2,900 g of purified water, and the temperature of the resulting aqueous solution was controlled to about 60°C. Then, in the obtained solution, a melted liquid formed by heating 400 g of the same type of CoQ₁₀ used in Example 1 to about 60°C to melt was dispersed. The resultant was then emulsified using a homogenizer (4,000 rpm, 5 minutes), and then passed through a high pressure homogenizer twice at a processing pressure of 750 kg/cm², as a result of which a fine and homogeneous emulsion was obtained.
Subsequently, the obtained emulsion was spray dried using the same spray dryer used in Example 5 with an inlet temperature of 170°C and an outlet temperature of 110°C, as a result of which an orange colored, highly water dispersible powder containing 40% by mass of CoQ₁₀ was obtained.
The mass ratio of each of the components in this working Example was such that poorly water soluble component (CoQ₁₀) : protective colloid polymer (gum arabic) : saponin (quillaja saponin) = 100 : 62.5 : 9.4.

### [Example 7]

15 g of the same type of HPC used in Example 3, 5 g of the same type of quillaja saponin used in Example 1, and 163.75 g of β-cyclodextrin (manufactured by Ensuiko Sugar Refining Co., Ltd. under the trade name of IsoElite P) were dissolved in 180 g of purified water at a normal temperature. Subsequently, 20 g of the same type of vitamin E used in Example 3 was added and mixed, and the resulting mixture was then passed through a high pressure homogenizer twice at a processing pressure of 500 kg/cm² while cooling, as a result of which a fine and homogeneous emulsion was obtained.
Subsequently, the obtained emulsion was spray dried using the same spray dryer used in Example 5 with an inlet temperature of 170°C and an outlet temperature of 110°C, as a result of which a pale yellowish white colored, highly water dispersible powder containing 10% by mass of vitamin E was obtained.
The mass ratio of each of the components in this working Example was such that poorly water soluble component (vitamin E) : protective colloid polymer (HPC) : saponin (quillaja saponin) = 100 : 75 : 6.25.

### [Comparative Example 1]

12 g of the same type of gum arabic used in Example 1 was dissolved in 50 g of purified water heated to about 60°C. Subsequently, while maintaining the temperature, 10 g of the same type of CoQ₁₀ used in Example 1 was added and mixed, and the resulting mixture was then passed through a high pressure homogenizer twice at a processing pressure of 750 kg/cm², as a result of which an emulsion was obtained.
Then, by using the same fluidized bed granulator used in Example 1, 78 g of the same type of dextrin used in Example 1 was made to flow in the fluidized bed and the obtained emulsion was sprayed thereto, as a result of which an orange colored matter (powder) was obtained which was a mixture of powdery and granular forms.
The mass ratio of each of the components in this Comparative Example was such that poorly water soluble component (CoQ₁₀) : protective colloid polymer (gum arabic) : saponin = 100 : 120 : 0.

### [Comparative Example 2]

5 g of the same type of HPC used in Example 3 was dissolved in 165 g of purified water at a normal temperature. Subsequently, 20 g of the same type of vitamin E used in Example 3 was added and mixed, and the resulting mixture was then passed through a high pressure homogenizer twice at a processing pressure of 500 kg/cm² while cooling, as a result of which an emulsion was obtained.
Then, by using the same fluidized bed granulator used in Example 1, 75 g of the same type of dextrin used in Example 1 was made to flow in the fluidized bed and the obtained emulsion was sprayed thereto, as a result of which a pale yellowish white colored matter (powder) was obtained which was a mixture of powdery and granular forms.
The mass ratio of each of the components in this Comparative Example was such that poorly water soluble component (vitamin E) : protective colloid polymer (HPC) : saponin =100 : 25 : 0.

### [Comparative Example 3]

80 g of the same type of quillaja saponin used in Example 1, 15 g of the same type of malic acid (manufactured by Fuso Chemical Co., Ltd. under the trade name of Malic Acid Fuso) used in Example 2, and 445 g of the same type of β-cyclodextrin used in Example 5 were dissolved in 3,000 g of purified water, and the temperature of the resulting aqueous solution was controlled to be about 60°C. Then, in the obtained solution, a melted liquid formed by heating 400 g of the same type of CoQ₁₀ used in Example 1 and 120 g of polyglycerol monolaurate having no protective colloid effect (manufactured by Riken Vitamin Co., Ltd. under the trade name of Poem J-0021) to about 60°C to melt was dispersed. The resultant was then emulsified using a homogenizer (4,000 rpm, 5 minutes), and then passed through a high pressure homogenizer twice at a processing pressure of 750 kg/cm², as a result of which an emulsion was obtained.
Subsequently, the obtained emulsion was spray dried using the same spray dryer used in Example 5 with an inlet temperature of 170°C and an outlet temperature of 80°C, as a result of which an orange colored powder was obtained.
The mass ratio of each of the components in this Comparative Example was such that poorly water soluble component (CoQ₁₀) : protective colloid polymer : saponin (quillaja saponin) = 100 : 0 : 5.

### [Comparative Example 4]

10 g of the same type of quillaja saponin used in Example 1 and 177.5 g of the same type of β-cyclodextrin used in Example 7 were dissolved in 160 g of purified water at a normal temperature. Subsequently, 20 g of the same type of vitamin E used in Example 3 was added and mixed, and the resulting mixture was then passed through a high pressure homogenizer twice at a processing pressure of 500 kg/cm², as a result of which an emulsion was obtained.
Subsequently, the obtained emulsion was spray dried using the same spray dryer used in Example 5 with an inlet temperature of 170°C and an outlet temperature of 110°C, as a result of which a pale yellowish white colored powder was obtained.
The mass ratio of each of the components in this Comparative Example was such that poorly water soluble component (vitamin E) : protective colloid polymer : saponin (quillaja saponin) = 100 : 0 : 12.5.

### <Test Example>

0.5 g of the powder obtained in the respective Examples and Comparative Examples was respectively added to 50 ml of water and stirred with a spatula and the emulsified states immediately after the stirring, 30 minutes after the stirring, 1 hour after the stirring, 24 hours after the stirring, and 7 days after the stirring were visually observed. The results are shown in Table 1. Evaluations were represented by assigning grades A, B, C, and D based on the following criteria.
A: Homogeneously dispersed state was observed, with no floating, separation, or precipitation of oil phase.
B: Homogeneously dispersed state was observed, although it was observed that a trace amount of oil phase was floated, separated, or precipitated.
C: Although a dispersed state was maintained, it was observed that a slight amount of oil phase was floated, separated, or precipitated.
D: Hardly any dispersion was observed, while it was observed that the solution was separated into two or more layers, or a large amount of oil phase was floated or precipitated.

**[Table 1]**

| | Immediately after stirring | After 30 minutes | After 1 hour | After 24 hours | After 7 days |
|---|---|---|---|---|---|
| Example 1 | A | A | A | A | B |
| Example 2 | A | A | A | A | B |
| Example 3 | A | A | A | A | B |
| Example 4 | A | A | A | A | B |
| Example 5 | A | A | A | B | B |
| Example 6 | A | A | A | A | B |
| Example 7 | A | A | A | A | A |
| | | | | | |
| Comparative Example 1 | C | D | D | D | D |
| Comparative Example 2 | C | D | D | D | D |
| Comparative Example 3 | A | B | B | D | D |
| Comparative Example 4 | A | B | B | B | D |

As is apparent from Table 1, the highly water dispersible powders obtained in the respective Examples exhibited a high water dispersibility when added to water, and the results showed that the high water dispersibility remained stable over time. On the other hand, the end products obtained in the respective Comparative Examples exhibited either unsatisfactory water dispersibility from immediately after the stirring, or a deterioration of the water dispersibility over time, even in the case that it exhibits high water dispersibility immediately after the stirring.

### INDUSTRIAL APPLICABILITY

According to the present invention, a highly water dispersible powder can be provided which exhibits a high water dispersibility in spite of the inclusion of a poorly water soluble component, and maintains the high water dispersibility stably over time. As a result, the highly water dispersible powder is easily added to food products such as supplements and health foods, and thus can be used in a wide variety of applications.

## Claims

1. A highly water dispersible powder comprising:
a poorly water soluble component;
a water soluble polymer having a protective colloid effect; and
a saponin.

2. The highly water dispersible powder according to Claim 1,
wherein an amount of the water soluble polymer having a protective colloid effect is within the range of 15 to 200 parts by mass, and an amount of the saponin is within the range of 4 to 30 parts by mass, relative to 100 parts by mass of the poorly water soluble component.

3. A method of producing a highly water dispersible powder comprising:
emulsifying a poorly water soluble component in an aqueous solvent under the presence of a water soluble polymer having a protective colloid effect and a saponin, thereby preparing an emulsion; and
drying the emulsion.
